**Europäisches Patentamt**

**European Patent Office**

**Office européen des brevets**

(19)

(11) Veröffentlichungsnummer: **0 017 887 B1**

(12) # EUROPÄISCHE PATENTSCHRIFT

(45) Veröffentlichungstag der Patentschrift:
27.03.85

(51) Int. Cl.⁴: **C 07 D 251/40,** C 07 D 251/64,
C 08 G 12/30, C 08 G 12/32

(21) Anmeldenummer: **80101849.0**

(22) Anmeldetag: **08.04.80**

(54) **Verfahren zur Herstellung von verätherten Methylolmelaminen.**

(30) Priorität: **14.04.79 DE 2915315**

(43) Veröffentlichungstag der Anmeldung:
**29.10.80 Patentblatt 80/22**

(45) Bekanntmachung des Hinweises auf die Patenterteilung:
**27.03.85 Patentblatt 85/13**

(84) Benannte Vertragsstaaten:
**BE CH DE FR GB IT LI NL SE**

(56) Entgegenhaltungen:
**AT - B - 163 630
AT - B - 190 518
AT - B - 199 657
AT - B - 250 981
CH - A - 432 839
DE - A - 2 005 166
DE - A - 2 430 899
DE - A - 2 516 349
DE - A - 2 716 006
DE - B - 1 015 438
DE - B - 1 218 456
DE - B - 1 232 130
DE - C - 949 740
DE - C - 970 453
GB - A - 809 662
US - A - 3 915 973**

(73) Patentinhaber: **CASSELLA Aktiengesellschaft, Hanauer
Landstrasse 526, D-6000 Frankfurt am Main 61 (DE)**

(72) Erfinder: **Michel, Walter, Dr., Fuldaer Strasse 27,
D-6000 Frankfurt am Main 61 (DE)**
Erfinder: **Hönel, Hans, Dr., Meerholzer Strasse 42,
D-6000 Frankfurt am Main 61 (DE)**
Erfinder: **Schön, Manfred, Dr., Saalburgring 3,
D-6051 Dudenhofen (DE)**
Erfinder: **Kaiser, Wolfgang, Kolpingstrasse 30,
D-6450 Hanau 8 (DE)**

(74) Vertreter: **Urbach, Hans-Georg, Dr. et al, Hanauer
Landstrasse 526, D-6000 Frankfurt am Main 61 (DE)**

(56) Entgegenhaltungen: (Fortsetzung)
**Die Akte enthält technische Angaben, die nach dem
Eingang der Anmeldung eingereicht wurden und die
nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die DE-A-2 516 349 betrifft ein Verfahren zur Herstellung von mit Alkanolen verätherten Methylol-Aminotriazinen mit einem analytischen Mittelwert von 0,7 n bis 2 n Methylol-Gruppen pro Mol des Aminotriazins, die zu 30—60% veräthert sind, wobei n die Zahl der Amino-Gruppen des Aminotriazins ist, bei dem ein Aminotriazin mit Formaldehyd und einem Alkanol bzw. mit einem Alkanolgemisch bei einem pH-Wert zwischen 3 und 6,5 bei Temperaturen zwischen 80 und 130°C unter erhöhtem Druck umgesetzt wird.

Verätherte Methylol-Aminotriazine haben im Laufe der industriellen Entwicklung der letzten Zeit immer mehr an Bedeutung gewonnen. Sie werden in zahlreichen Anwendungsbereichen in ständig steigenden Mengen eingesetzt. So sind sie beispielsweise als Aminoplastvernetzer in wasserlöslichen und in lösungsmittellöslichen Lackkombinationen, als Vernetzer für hydroxylgruppenhaltige Latices und Dispersionsbinder, zur Herstellung von Preßmassen, als Bestandteile wasserfester Klebstoffe für Furnierleimung, bei der Herstellung von Schichtstoffen, Umleimern und Oberflächenformung von Plattenmaterial unentbehrlich. Auch in der Papierindustrie finden alkanolverätherte, hauptsächlich jedoch methylverätherte Methylol-Aminotriazine zur Herstellung trocken- und naßreißfester Oberflächen und zur Verbesserung von Papierstrichen, insbesondere zu deren Wasserfestmachung, Verwendung, und in der Textilindustrie werden derartige Produkte als Hochveredlungsmittel eingesetzt.

Für die Anwendung der alkanolverätherten Methylol-Aminotriazine in allen oben genannten Bereichen sind Oberflächenhärte, Kratzfestigkeit, Trockenhitzebeständigkeit, Resistenz gegen Wasserdampf, Haftfestigkeit und Elastizität der durch einen durch Säure- und/oder Hitzeeinwirkung bewirkten Härtungsprozeß daraus entstehenden ausgehärteten aminoplastvernetzten Überzüge und Oberflächenfilme besonders erwünscht.

Die Herstellung der verätherten Methylol-Aminotriazine erfolgt durch Umsetzung des Aminotriazins mit Formaldehyd und dem zur Verätherung benutzten Alkanol in Gegenwart einer Säure. Die Reaktion wurde bisher stets im Temperaturbereich zwischen normaler Zimmertemperatur und dem Siedepunkt der Reaktionsmischung durchgeführt.

So ist aus der DE-A-2 005 166 ein Verfahren bekannt zur Herstellung wasserlöslicher, verätherter Methylolmelamine, bei welchem man in einer ersten Stufe 1 Mol Melamin mit 2 bis 6 Mol Paraformaldehyd in 0,4 bis 1,2 Mol eines 2- oder 3wertigen Alkohols mit insgesamt 2 bis 6 Kohlenstoffatomen, wobei ein Teil des Polyalkohols durch den in der zweiten Stufe zur Verätherung verwendeten niedrigen, einwertigen, aliphatischen Alkohol ersetzt werden kann, in Gegenwart von Alkali am Rückfluß erhitzt, bis eine opaleszente bis klare Lösung entsteht und unmittelbar anschließend in der zweiten Stufe nach Zusatz von überschüssigem, niedrigem, einwertigem, aliphatischem Alkohol unter Kochen am Rückfluß sauer veräthert.

Ein ähnliches, bei Normaldruck arbeitendes Verfahren, bei dem der Formaldehyd auch vorzugsweise in Form des teuren Paraformaldehyd eingesetzt wird, ist aus der AT-B-190 518 bekannt. Bei diesem Verfahren erfolgt eine Umsetzung von Melamin mit Formaldehyd in einer Wasser und den aliphatischen Alkohol enthaltenden Mischung bei einer Temperatur zwischen 50°C und Rückflußtemperatur von 80°C und bei einem pH-Wert zwischen 7 und 12, wobei das Mol-Verhältnis von Alkohol zu Wasser zwischen etwa 5 : 1 und 0,5 : 1, das Mol-Verhältnis von Alkohol zu Formaldehyd zwischen etwa 0,4 : 1 und 5 : 1 und das Mol-Verhältnis von Melamin zu Formaldehyd zwischen etwa 1 : 1, und 1 : 6 liegt und wobei hierauf der Reaktionszone weitere Anteile des Alkohols in solchen Mengen zugefügt werden, daß das Mol-Verhältnis zwischen der insgesamt eingebrachten Alkoholmenge und dem eingebrachten Wasser mindestens etwa 2 : 1 beträgt. Auch dieses Verfahren verläuft im wesentlichen in zwei Stufen:

(A) Umsetzung von Melamin mit Formaldehyd unter alkalischen Bedingngen in einer festgelegten Menge Wasser und Verätherungsalkohol enthaltenden Mischung.

(B) Alkylierung unter sauren Bedingungen unter weiterer Zugabe von Alkohol, sofern nötig.

Diese herkömmlichen Verfahren zeigen jedoch schwerwiegende Nachteile. Erstens ist die Raumzeitausbeute unbefriedigend, insbesondere dann, wenn verätherte Methylol-Aminotriazine mit geringem Methylolierungsgrad hergestellt werden sollen. Hierbei werden schnell Reaktionszeiten erreicht, die die bekannten Verfahren völlig unwirtschaftlich werden lassen. Versucht man in diesem Fall die Reaktionsgeschwindigkeit durch Erhöhung der Säurekonzentration in den Ansätzen zu erhöhen, so muß nach Ablauf der Reaktion und der Neutralisation der Säure das entstandene Salz aus den Vorkondensaten entfernt werden, wozu eine Filtrationsoperation erforderlich ist, die den Zeitgewinn, der durch die höhere Reaktionsgeschwindigkeit erzielt wurde, wiederaufhebt. Dieser Nachteil wirkt sich umso unangenehmer aus, als besonders Harze mit niedrigem Formaldehydanteil bei ihrer Verwendung auf dem Schichtstoffsektor wesentliche Vorteile bieten: Sie zeigen höhere Reaktivität, und sie spalten erheblich weniger Formaldehyd bei der Aushärtung ab, wodurch die Anwendung wesentlich erleichtert wird. Ein weiterer Nachteil der bekannten Verfahren besteht darin, daß es kaum gelingt, niedrig methylolierte Vorkondensate herzustellen, die lagerstabil sind, d. h. die bei längerer Lagerung nicht zur Kristallisation neigen.

Nach dem Verfahren der DE-A-216 349 lassen sich diese Nachteile vermeiden, wenn zur Herstellung von mit Alkanolen verätherten Methylol-Aminotriazinen mit einem analytischen Mittelwert von 0,7 n bis 2 n Methylolgruppen pro Mol des Aminotriazins, die zu 30 bis 60% veräthert sind, wobei n die Zahl der Aminogruppen des Aminotriazins ist, ein Aminotriazin mit 0,7 n bis 3 n Mol Formaldehyd, 2 n bis 10 n Mol eines Alkanols oder eines Gemisches von Alkanolen mit 1—8 Kohlenstoffatomen, deren Kohlenstoffkette, sofern sie mehr als 2 Kohlenstoffatome hat, auch durch ein Sauerstoffatom unterbrochen sein kann, und 0 bis 5 n Mol Wasser pro Mol des Aminotriazins in Gegenwart einer anorganischen oder organischen Säure bei einem pH-Wert zwischen 3 und 6,5, 0,2 bis 20 Minuten lang unter erhöhtem Druck auf 80—130° C erwärmt wird. Vorzugsweise werden pro Mol des Aminotriazins 0,77 n—3 n Mol Formaldehyd und 2,5 n—7 n Mol des Alkanols oder Alkanolgemischs verwendet. Zur Einstellung bestimmter Kondensationsgrade wird die zugesetzte Wassermenge vorzugsweise im Bereich von 0 bis 3 n Mol pro Mol des Aminotriazins variiert. Zur Herstellung besonders niedrig kondensierter Produkte wird vorzugsweise wasserfrei gearbeitet. Aminotriazine, die vorteilhaft nach diesem Verfahren umgesetzt werden können sind Benzoguanamin und insbesondere Melamin.

Es wurde nun gefunden, daß man zu Produkten mit weiter verbesserten Eigenschaften, insbesondere weiter verbesserter Verträglichkeit mit organischen Binde- und Verdünnungsmitteln, niedrigerer Viskosität, hervorragender Reaktivität bei weiter erhöhter Lagerbeständigkeit in Gegenwart von vernetzbaren Bindemitteln und zu Produkten mit einem analytischen Mittelwert von 1,8 bis 6 Methylolgruppen pro Melaminmolekül, die zu 30—60% veräthert sind, gelangt, wenn man Melamin mit Formaldehyd und Alkohol als Verätherungsagens in einem Zweistufenprozeß zur Reaktion bringt, wobei die Reaktion in der ersten Stufe in alkalischem Medium bei 60—90° C und gegebenenfalls in Gegenwart eines Teils des Verätherungsagens erfolgt und in der zweiten Stufe der Rest des zum Einsatz vorgesehenen Verätherungsagens zugesetzt und das Reaktionsmedium bei pH 3—8 gehalten wird, wobei man als Verätherungsagens Methanol oder eine Mischung von Methanol mit bis zu 20 Gew.-% eines Glycols mit 2 bis 4 C-Atomen oder eines Polyethylenglycols mit 2 bis 4 Ethylenglycol-Einheiten oder eines Monoalkyl-(C₁—C₄)-äthers der genannten Glycole und Polyglycole einsetzt und

a) in der 1. Stufe 1 Mol Melamin mit 2,1—9 Molen Formaldehyd 0—15 Molen Wasser und 0—30 Gew.-% der zum Einsatz vorgesehenen 6—30 Mole des Verätherungsagens gegebenenfalls bei Anwesenheit eines unter dem Reaktionsbedingungen inerten organischen Lösungsmittels 1 bis 30 Minuten bei pH 8—11 zur Reaktion bringt und

b) in der 2. Stufe die Reaktionsmischung nach Zugabe des restlichen Verätherungsagens während 0,2 bis 20 Minuten bei 80—130° C unter einem Druck von mindestens 0,1013 bar Überdruck (0,1 atü) gehalten wird.

Diole mit 2 bis 4 C-Atomen, und Polyäthylenglycole und deren Monoalkyläther, die für das erfindungsgemäße Verfahren in Betracht kommen, sind Äthylenglycol; 1,2- und 1,3-Propandiol; 1,2-1,3- oder 1,4-Butandiol; Diäthylenglycol (HO—CH₂CH₂O—CH₂CH₂—OH), Triäthylenglycol H(OCH₂CH₂—)₃—OH und Tetraethylenglycol H(OCH₂CH)₄—OH, Glycol-mono-methyl-, -äthyl-, -propyl- oder -butyläther, Methoxypropanol, Methoxyisopropanol, Äthoxypropanol, Methoxy- bzw. Äthoxybutanol, Methoxy- bzw. Äthoxyisobutanol, Diäthylenglycol-mono-methyl-, -äthyl-, propyl- oder butyläther, Triäthylenglycolmono-butyläther.

Bevorzugt sind Äthylenglycol, die Propandiole und Di- und Triäthylenglycol.

Der Einsatz von Mischungen von Methanol mit bis zu 20 Gew.-% der oben genannten Glycole, Polyole bzw. deren Monoäthern bei der neuen Arbeitsweise gestattet noch eine Erweiterung der Variationsmöglichkeiten der speziellen, vorteilhaften Eigenschaften der Verfahrensprodukte und damit eine zusätzliche Ausweitung ihrer vorteilhaften Anwendungsmöglichkeiten.

Nach dem Zusatz des gesamten Methanols bzw. Verätherungsgemisches erfolgt im wesentlichen die Verätherung der im ersten, drucklos durchgeführten Reaktionsschritt entstandenen Methylolmelamine. Der hierbei bevorzugte Temperaturbereich liegt zwischen 85 und 115° C. Da die Siedetemperatur des Verätherungsgemisches bei Normaldruck im allgemeinen unter 90° C liegt, wird in einer druckfest verschlossenen Apparatur gearbeitet. Auf jeden Fall wird die Temperatur so weit gesteigert, daß in der Apparatur mindestens ein Überdruck von 0,1 Atmosphären entsteht. Die Verätherung mit der Gesamtmenge des Alkanols bzw. Alkanolgemischs wird bei einem pH-Wert von 3 bis 8, vorzugsweise 3 bis 7, ganz besonders bevorzugt zwischen 6 und 7,0 ausgeführt. Dieser pH-Wert kann durch Zusatz einer starken anorganischen oder organischen Säure in Mengen von 0,1 bis 1 pro Mille zum Reaktionsansatz erzielt werden. Säuren, die für das erfindungsgemäße Verfahren eingesetzt werden können, sind anorganische Säuren, wie z. B. Schwefelsäure, Salpetersäure, Brom-, Chlor-, Fluor- oder Jodwasserstoffsäure, sowie Verbindungen, aus denen diese unter den beanspruchten Reaktionsbedingungen freigesetzt werden, oder organische Säuren vergleichbarer Säurestärke, wie z. B. Sulfonsäuren, Ameisensäure oder Halogenessigsäuren.

Prinzipiell besteht auch die Möglichkeit, den notwendigen pH-Wert durch Zusatz schwächerer Säuren einzustellen, jedoch ist es vorteilhaft, mit stärkeren Säuren zu arbeiten.

Das erfindungsgemäße Verfahren kann gegebenenfalls auch in Gegenwart inerter organischer Lösungsmittel ausgeführt werden. Als inerte organische Lösungsmittel sind beispielsweise geeignet: Niedere Aromaten, aliphatische Äther, subst. Amide wie Dimethylformamid, tert. Alkohole wie tert. Butanol.

Das erfindungsgemäße Verfahren verbindet die Vorteile des Verfahrens der DE-A-2 516 349 mit einer noch weiter verbesserten Qualität der Verfahrensprodukte in Bezug auf Verträglichkeit mit organischen Binde- und Verdünnungsmitteln, die einen Einsatz in noch stärker oleophilen Bindemittelsystemen ermöglicht, niedrigere Viskosität und höherer Reaktivität bei gleichzeitig hoher Haltbarkeit auch in Gegenwart vernetzbarer Bindemittel.

Das Verfahren ist noch flexibler und die Eigenschaften der Produkte können dadurch noch besser den Praxiserfordernissen angepaßt werden. So können Produkte mit weiter verbesserter Eigenvernetzbarkeit erhalten werden, die als Alleinbindemittel beispielsweise zur Herstellung von gebundenen Wirrfaservliesen hervorragend geeignet sind.

Die Prozentangaben in den folgenden Beispielen sind Gewichtsprozente. Der in den Beispielen angegebene Festkörpergehalt wird bestimmt, indem man eine Probe von ca. 2 g Lösung bei 120°C 1 Stunde lang trocknet und den Rückstand bestimmt. Die zur Angabe der Viskosität in den Beispielen genannten Auslaufzeiten sind Auslaufzeiten nach 4/DIN 53 211, d. h. sie wurden mittels DIN-Auslaufbechern gemäß DIN 53 211 bestimmt.

Die Vergleichsbeispiele veranschaulichen Harze gleicher oder sehr naheliegender Zusammensetzung (bezogen auf Ausgangsmaterialien) wie die des betreffenden Beispiels, jedoch hergestellt nach dem Verfahren der DE-A-2 516 349.

### Beispiel 1

2400 g Melamin, 5860 g 39%ige wäßrige Formaldehydlösung und 10 g 50%iges Natriumhydroxid werden in einem druckfest verschließbaren Gefäß bei geöffnetem Druckverschluß am Rückflußkühler auf 75°C erwärmt und 15 Minuten bei dieser Temperatur gerührt. Das zunächst ungelöste Melamin geht im Verlauf der Reaktion vollständig in Lösung.

Die klare Lösung wird auf 45 bis 40°C abgekühlt und mit 10440 g Methanol und 11 g 65%iger Salpetersäure versetzt, wobei der pH-Wert auf 6,5 absinkt.

Das Reaktionsgefäß wird nun druckfest verschlossen, auf 85—87°C erwärmt und 15 Minuten bei dieser Temperatur gerührt, wobei sich ein Innendruck von 1,5 bar einstellt.

Anschließend wird durch Zusatz von 13 ml 27%igem Natriumhydroxid wieder ein pH-Wert von 9,5 eingestellt und bei 70°C unter vermintem Druck auf einen Festkörpergehalt von 75 Gew.-% eingedampft.

Man erhält eine klare Harzlösung mit einer Viskosität von 101 DIN sec, bestimmt bei 20°C im 4 mm Auslaufbecher gemäß DIN 53211. Wasserverdünnbarkeit: 1 : ∞ Isopropanolverdünnbarkeit: 1 : ∞.

Die Abkühlung des Ansatzes vor der Zugabe der 10440 g Methanol kann unterbleiben, wenn die Zugabe durch den Rückflußkühler nicht zu schnell erfolgt.

### Vergleichsbeispiel 1a

5150 g Methanol, 2930 g 39%ige wäßrige Formaldehydlösung, 6 ml konzentrierte Salpetersäure und 1200 g Melamin werden in einem Autoklaven gemischt. Der pH-Wert der Mischung beträgt 5,5. Nach druckfestem Verschließen des Gefäßes wird unter Rühren innerhalb von 5 Minuten auf 90°C erwärmt, wobei sich ein Druck von 1,7 bis 1,8 bar einstellt. Bei dieser Temperatur wird 15 Minuten weitergerührt. Nach Ablauf dieser Zeit werden 15 ml 27%ige Natronlauge eingespritzt und der Autoklav sofort abgekühlt. Das Reaktionsprodukt wird anschließend durch Eindampfen bei ca. 30 mbar und 70°C auf einen Festkörpergehalt von 75 Gew.-% eingeengt. Man erhält eine klare Harzlösung mit einer Viskosität von 255 DIN-sec, bestimmt gemäß DIN 53211 im 4-mm-Auslaufbecher. Wasserverdünnbarkeit: 1 : 2; Isopropanolverdünnbarkeit: 1 : 2,3.

### Beispiel 2

1260 g Melamin, 5390 g 39%ige wäßrige Formaldehydlösung und 60 g 50%iges Natriumhydroxid werden in einem druckfest verschließbaren Gefäß bei geöffnetem Druckverschluß am Rückflußkühler auf 75°C erwärmt und 15 Minuten bei dieser Temperatur kräftig gerührt, wobei das zunächst ungelöste Melamin im Verlauf der Reaktion vollständig in Lösung geht, und gegen Ende der Reaktion scheidet sich eine Ausfällung von Methylolmelamin ab. Die Suspension wird auf 45 bis 40°C abgekühlt und mit 5400 g Methanol und 31 ml 65%iger Salpetersäure versetzt, wobei der pH-Wert auf 6,6 absinkt.

Das Reaktionsgefäß wird nun druckfest verschlossen, auf 85—87°C erwärmt und 15 Minuten bei dieser Temperatur gerührt, wobei sich ein Innendruck von 1,4 bar einstellt.

Anschließend wird durch Zusatz von 10 ml 27%iger Natriumhydroxidlösung wieder ein pH-Wert von 10 eingestellt und bei 70°C unter vermindertem Druck auf einen Festkörpergehalt von 73,5 Gew.-% eingedampft.

Man erhält eine klare Harzlösung mit einer Viskosität von 90 DIN-sec, bestimmt bei 20°C im 4-mm-Auslaufbecher gemäß DIN 53211. Wasserverdünnbarkeit: 1 : ∞; Isopropanolverdünnbarkeit: 1 : ∞.

Die Abkühlung des Ansatzes vor der Zugabe der 5400 g Methanol kann unterbleiben, wenn die Zugabe durch den Rückflußkühler nicht zu schnell erfolgt.

### Vergleichsbeispiel 2a

1500 g Methanol, 1325 g 39%ige wäßrige Formaldehydlösung, 316 g Melamin und 2 g p-Toluolsulfonsäure, gelöst in 25 g Methanol, werden in einem Autoklaven gemischt. Nach druckfestem Verschließen des Gefäßes wird unter Rühren auf 90°C erwärmt, wobei sich ein Druck von 1,5 bar einstellt. Bei dieser Temperatur wird 5 Minuten weitergerührt. Nach Ablauf dieser Zeit werden 35 ml 10%ige Natriumhydrogencarbonat-Lösung eingespritzt und der Autoklav sofort abgekühlt. Das Reaktionsprodukt wird anschließend durch Eindampfen bei ca. 30 mbar und 70°C auf einen Festkörpergehalt von 75 Gew.-% eingeengt.

Nach dem Abkühlen wird die eingeengte Lösung fest. Sie kann daher, im Gegensatz zu der gemäß Beispiel 2 hergestellten Harzlösung, nicht mehr bei Raumtemperatur verarbeitet werden.

### Beispiel 3

63,0 kg Melamin, 204 kg 39%ige wäßrige Formaldehydlösung, 45 kg Methanol und 343 g 50%ige Natriumhydroxidlösung werden in einem druckfest verschließbaren Gefäß bei geöffnetem Druckverschluß am Rückflußkühler auf 75°C erwärmt und 10 Minuten bei 75 bis 65°C kräftig gerührt. Das zunächst ungelöste Melamin geht im Verlauf der Reaktion vollständig in Lösung, und es scheidet sich Methylolmelamin als weißer Festkörper aus der Lösung aus.

Die Suspension wird auf 45 bis 40°C abgekühlt und mit 280 kg Methanol und 350 ml 65%iger Salpetersäure versetzt, wobei der pH-Wert auf 6,5 absinkt.

Das Reaktionsgefäß wird nun druckfest verschlossen, auf 85–87°C erwärmt und es wird 15 Minuten bei dieser Temperatur gerührt, wobei sich ein Innendruck von 0,85 bar einstellt.

Anschließend wird durch Zusatz von 400 ml 27%igem Natriumhydroxid wieder ein pH-Wert von 10,5 eingestellt und bei 70°C unter vermindertem Druck auf einen Festkörpergehalt von 75 Gew.-% eingedampft.

Man erhält eine klare Harzlösung mit einer Viskosität von 116-DIN-sec, bestimmt bei 20°C im 4-mm-Auslaufbecher gemäß DIN 53211. Wasserverdünnbarkeit: 1 : ∞; Isopropanolverdünnbarkeit: 1 : ∞.

Die Abkühlung des Ansatzes vor der Zugabe der 280 kg Methanol kann unterbleiben, wenn die Zugabe durch den Rückflußkühler nicht zu schnell erfolgt.

### Vergleichsbeispiel 3a

6825 g Methanol, 4284 g 39%ige wäßrige Formaldehydlösung, 1,4 g konzentrierte Salpetersäure und 1223 g Melamin werden in einem Autoklaven gemischt. Der pH-Wert der Mischung beträgt 6,2. Nach druckfestem Verschließen des Gefäßes wird unter Rühren auf 80°C erwärmt, wobei sich ein Druck von 1,0 bar einstellt. Bei dieser Temperatur wird 35 Minuten weitergerührt. Nach Ablauf dieser Zeit werden 2 ml 27%ige Natronlauge eingespritzt und der Autoklav sofort abgekühlt. Das Reaktionsprodukt wird anschließend durch Eindampfen bei ca. 30 mbar und 70°C auf einen Festkörpergehalt von 75 Gew.-% eingeengt.

Nach dem Abkühlen wird die eingeengte Lösung fest. Sie kann daher, im Gegensatz zu der gemäß Beispiel 1 hergestellten Harzlösung, nicht mehr bei Raumtemperatur verarbeitet werden. Beim Erwärmen auf 80°C erhält man jedoch eine klare Lösung mit einer Viskosität von 230-DIN-sec. im 4-mm-DIN-Auslaufbecher, einer Wasserverdünnbarkeit von 1 : ∞ und einer Isopropanolverdünnbarkeit von 1 : ∞.

### Beispiel 4

63 kg Melamin, 216 kg 39%ige wäßrige Formaldehydlösung, 92 kg Methanol und 390 g 50%iges Natriumhydroxid werden in einem druckfest verschließbaren Gefäß bei geöffnetem Druckverschluß am Rückflußkühler auf 75°C erwärmt und 15 Minuten bei dieser Temperatur gerührt. Das zunächst ungelöste Melamin geht im Verlauf der Reaktion vollständig in Lösung.

Die klare Lösung wird auf 45 bis 40°C abgekühlt, wobei sich ein Niederschlag von Methylolmelamin ausscheidet, und mit 217 kg Methanol und 1,5 l 65%iger Salpetersäure versetzt.

Das Reaktionsgefäß wird nun druckfest verschlossen, auf 85–87°C erwärmt und 15 Minuten bei dieser Temperatur gerührt, wobei sich ein Innendruck von 0,9 bar einstellt.

Anschließend wird durch Zusatz von ca. 800 ml 27%iger Natronlauge wieder ein pH-Wert von 10 eingestellt und bei 70°C unter vermindertem Druck auf einen Festkörpergehalt von 74 Gew.-% eingedampft.

Man erhält eine klare Harzlösung mit einer Viskosität von 118-DIN-sec., bestimmt bei 20°C im 4-mm-Auslaufbecher gemäß DIN 53211. Wasserverdünnbarkeit: 1 : ∞; Isopropanolverdünnbarkeit: 1 : ∞.

### Vergleichsbeispiel 4a

1500 g Methanol, 1156 g 39%ige wäßrige Formaldehydlösung, 316 g Melamin und 1 g p-Toluolsulfonsäure, gelöst in 25 ml Methanol, werden in einem Autoklaven gemischt. Nach druckfestem Verschließen des Gefäßes wird unter Rühren auf 100°C erwärmt, wobei sich ein Druck von 1,7 bar einstellt. Bei dieser Temperatur wird 9 Minuten weitergerührt. Nach Ablauf dieser Zeit werden 10 ml 10%ige Natriumhydrogencarbonat-Lösung eingespritzt und der Autoklav sofort abgekühlt. Das Reaktionsprodukt wird anschließend durch Eindampfen bei ca. 30 mbar und 70°C auf einen Festkörpergehalt von 75 Gew.-% eingeengt.

Die so erhaltene klare Harzlösung hat bei unbegrenzter Verdünnbarkeit in Wasser und Isopropanol eine Viskosität von 210-DIN-sec. im 4-mm-Auslaufbecher bei 20°C.

### Beispiel 5

1260 g Melamin, 3846 g 39%ige wäßrige Formaldehydlösung, 900 g Methanol und 6 g 50%iges Natriumhydroxid werden in einem druckfest verschließbaren Gefäß bei geöffnetem Druckverschluß am Rückflußkühler auf 75°C erwärmt und 10 Minuten bei dieser Temperatur gerührt. Die Mischung hat den pH-Wert 10, und das zunächst ungelöste Melamin geht im Verlauf der Reaktion vollständig in Lösung.

Die klare Lösung wird auf 45 bis 40°C abgekühlt und mit 5266 g Methanol, 468 g Triäthylenglycol und 45 g 65%iger Salpetersäure versetzt, wobei der pH-Wert auf 6,5 absinkt. Das Reaktionsgefäß wird nun druckfest verschlossen auf 85—87°C erwärmt und 15 Minuten bei dieser Temperatur gerührt wobei sich ein Innendruck von 1,5 bar einstellt. Anschließend wird durch Zusatz von 6,5 g 55%igem Natriumhydroxid wieder ein pH-Wert von 10 eingestellt und bei 70°C unter vermindertem Druck auf einem Festkörpergehalt von 75 Gew.-% eingedampft. Man erhält eine klare Harzlösung mit einer Viskosität von 95-DIN-sec., bestimmt bei 20°C im 4-mm-Auslaufbecher gemäß DIN 53211. Wasserverdünnbarkeit: 1 : ∞; Isopropanolverdünnbarkeit: 1 : ∞. Die Abkühlung des Ansatzes vor der Zugabe der 5266 g Methanol kann unterbleiben, wenn die Zugabe durch den Rückflußkühler nicht zu schnell erfolgt.

Unterläßt man die Zugabe der 468 g Triäthylenglycol vor der Verätherung, so erhält man ein Produkt, das bei sonst praktisch gleichen Eigenschaften eine deutlich verringerte Isopropanolverdünnbarkeit aufweist.

### Vergleichsbeispiel 5a

4362 g Methanol, 2740 g 39%ige wäßrige Formaldehydlösung, 8,8 g 27%ige Natronlauge, 330 g Triäthylenglycol und 900 g Melamin werden in einem Autoklaven gemischt. Der pH-Wert der Mischung beträgt 11. Nach druckfestem Verschließen des Gefäßes wird unter Rühren auf 85 bis 87°C erwärmt, wobei sich ein Druck von 1,2 bar einstellt. Bei dieser Temperatur wird 6,6 g 55%ige Salpetersäure eingespritzt und 10 Minuten weitergerührt. Nach Ablauf dieser Zeit werden 10 g 50%ige Natronlauge eingespritzt und der Autoklav sofort abgekühlt. Das Reaktionsprodukt wird anschließend durch Eindampfen bei ca. 30 m bar und 70°C auf einen Festkörpergehalt von 75 Gew.-% eingeengt. Nach dem Abkühlen wird die eingeengte Lösung fest. Sie kann daher, im Gegensatz zu der gemäß Beispiel 1 hergestellten Harzlösung nicht mehr bei Raumtemperatur verarbeitet werden.

Zu einer Substanz mit gleichem Verhalten gelangt man, wenn man die Formaldehydmenge auf 2200 g reduziert.

### Patentansprüche

1. Verfahren zur Herstellung von verätherten Methylolmelaminen mit einem analytischen Mittelwert von 1,8 bis 6 Methylolgruppen pro Melaminmolekül die zu 30—60% veräthert sind, bei welchem Melamin mit Formaldehyd und Alkohol als Verätherungsagens in einem Zweistufenprozeß zur Reaktion gebracht werden, wobei die Reaktion in der ersten Stufe in alkalischem Medium bei 60—90°C und ggf. in Gegenwart eines Teils des Verätherungsagens erfolgt, und in der zweiten Stufe der Rest des zum Einsatz vorgesehenen Verätherungsagens zugesetzt und das Reaktionsmedium bei pH 3—8 gehalten wird, dadurch gekennzeichnet, daß als Verätherungsagens Methanol oder eine Mischung von Methanol mit bis zu 20 Gew.-% eines Glycols mit 2 bis 4 C-Atomen oder eines Polyethylenglycols mit 2 bis 4 Ethylenglycol-Einheiten oder eines Monoalkyl($C_1$—$C_4$)äthers der genannten Glycole und Polyglycole eingesetzt wird und daß man

a) in der ersten Stufe 1 Mol Melamin mit 2,1—9 Molen Formaldehyd, 0—15 Molen Wasser und 0—30 Gew.-% der zum Einsatz vorgesehenen 6—30 Mole des Verätherungsagens gegebenenfalls bei Anwesenheit eines unter den Reaktionsbedingungen inerten organischen Lösungsmittels 1 bis 30 Minuten bei pH 8—11 zur Reaktion bringt und

b) in der zweiten Stufe die Reaktionsmischung nach Zugabe des restlichen Verätherungsagens während 0,2 bis 20 Minuten bei 80—130°C unter einem Druck von mindestens 0,1013 bar Überdruck (0,1 atü) gehalten wird.

2. Verfahren gemäß Anspruch 1, dadurch gekennzeichnet, daß nach Zusatz des gesamten Verätherungsagens bei einem pH-Wert zwischen 6 und 7,0 gearbeitet wird.

### Claims

1. Process for the preparation of etherified methylol-melamines which have, per melamine molecule, an analytically determined average of 1.8 to 6 methylol groups which are etherified to the extent of 30 to 60% in which process melamine is reacted in a two-step process with formaldehyde and alcohol as etherification agent, in which reaction the first step is carried out in an alkaline medium at 60 to 90°C and, if appropriate, in the presence of part of the etherification agent and in the second step the remainder of the etherifying agent envisaged is added and the reaction medium is maintained at pH 3 - 8, characterised in that the etherification agent used is methanol or a mixture of methanol with up to 20% by weight of a glycol having 2 to 4 C atoms or of a polyethyleneglycol having 2 to 4 ethyleneglycol units or of a monoalkyl ($C_1 - C_4$) ether of the mentioned glycols and polyglycols, and it that

a) in the first step 1 mole of melamine is reacted for 1 to 30 minutes at pH 8 - 11 with 2.1 to 9 moles of formaldehyde, 0 to 15 moles of water and 0 to 30% by weight of the envisaged 6 to 30 moles of etherification agent, if appropriate, in the presence of an organic solvent which is inert under the reaction conditions and

b) in the second step the reaction mixture, after addition of the remainder of the etherification agent, is maintained for 0.2 to 20 minutes at 80 - 130"C and under an excess pressure of at least 0.1013 bar (0.1 atmospheres gauge).

2. Process according to Claim 1, characterised in that after the addition of the complete etherification agent the process is continued at a pH value between 6 and 7,0.

## Revendications

1. Procédé pour préparer des méthylol-mélamines éthérifiées dont la teneur analytique moyenne en radicaux méthylols, par molécule de mélamine, est comprise entre 1,8 et 6 et dont le taux d'éthérification est compris entre 30 et 60%, procédé selon lequel on fait réagir la mélamine avec le formaldéhyde et l'alcool devant servir d'agent d'éthérification dans une méthode à deux étapes au cours de laquelle, dans la premiere étape, on effectue la réaction en milieu alcalin à une température de 60 à 90°C et éventuellement en présence d'une partie de l'agent d'éthérification et, dans la seconde étape, on ajoute le reste de la quantité prévue de l'agent d'éthérification et on maintient le milieu réactionnel à un pH de 3 à 8, procédé caractérisé en ce qu'on utilise, comme agent d'éthérification, le méthanol ou un mélange de méthanol avec jusqu'à 20% en poids d'un glycol contenant de 2 à 4 atomes de carbone ou d'un polyéthylène-glycol contenant de 2 à 4 motifs d'éthylène-glycol ou d'un éther mono-alkylique des glycols et polyglycols mentionnés dont la partie alkylique contient de 1 à 4 atomes de carbone et en ce que:

a) dans la première étape on fait réagir 1 mol de mélamine avec de 2,1 à 9 mol de formaldéhyde, de 0 à 15 mol d'eau et de 0 à 30% en poids des 6 à 30 mol de l'agent d'éthérification que l'on a prévu de mettre en jeu, éventuellement en présence d'un solvant organique inerte dans les conditions de la réaction, pendant une durée de 1 à 30 minutes, à un pH de 8 à 11, et

b) dans la seconde étape on maintient le mélange réactionnel, après y avoir ajouté le reste de l'agent d'éthérification, pendant 0,2 à 20 minutes à une température de 80 à 130"C et sous une pression effective d'au moins 0,1013 bar (0,1 atmosphère).

2. Procédé selon la revendication 1, caractérisé en ce que, après l'addition complète de l'agent d'éthérification on opère à un pH de 6 à 7,0.